# EUROPEAN PATENT SPECIFICATION

(11) **EP 3 524 695 B1**
(45) Date of publication and mention of the grant of the patent: **20.07.2022**
(21) Application number: 18156490.7
(22) Date of filing: 13.02.2018
(51) Int. Cl.: C12Q 1/6806, C12Q 1/6869

(54) **METHOD FOR THE ENRICHMENT OF GENOMIC REGIONS**
VERFAHREN ZUR ANREICHERUNG GENOMISCHER REGIONEN
PROCÉDÉ POUR ENRICHISSEMENT DE RÉGIONS GÉNOMIQUES

(43) Date of publication of application: 14.08.2019
(73) Proprietor: Personal Genomics S.r.l., 37136 Verona VR (IT)
(72) Inventor: DELLEDONNE, Massimo, 37136 Verona VR (IT)
(74) Representative: Trupiano, Federica

(56) References cited:
- WO-A1-2016/022833
- US-A1- 2015 203 907
- US-A1- 2015 211 054
- ÍTALOFARIA DO VALLE ET AL: "Optimized pipeline of MuTect and GATK tools to improve the detection of somatic single nucleotide polymorphisms in whole-exome sequencing data", BMC BIOINFORMATICS, BIOMED CENTRAL LTD, LONDON, UK, vol. 17, no. 12, 8 November 2016 (2016-11-08), pages 27-35, XP021237496, DOI: 10.1186/S12859-016-1190-7
- FERRARINI ALBERTO ET AL: "The Use of Non-Variant Sites to Improve the Clinical Assessment of Whole-Genome Sequence Data", PLOS ONE, vol. 10, no. 7, July 2015 (2015-07), XP002780529,

## Description

### Field of the invention

The present invention relates to the field of molecular biology and bioinformatics. In particular, the present invention relates to the field of targeted resequencing of specific nucleic acid sequences in a genome.

### Background of the invention

Mutation in the sequence of one or more genes may cause a great variety of diseases. Detection of mutations is currently performed by techniques such as real-time PCR, Sanger Sequencing, microarray hybridization and the so called Next Generation Sequencing (NGS).

In order to focus the sequence analysis to specific regions of interest (ROIs) of the genome, an enrichment step can be performed before the sequencing step to increase the amount of targeted sequences with respect to non-targeted sequences, i.e. to sequences of no interest. The term "targeted enrichment" generally refers to a procedure to selectively make specific sequences (for example, specific regions of interest of a genome) to be over-represented from a nucleic acid (e.g. DNA) sample before sequencing. These enrichment procedures allow the focusing of the sequencing to the sequences of interest, which have been enriched, i.e., increased in their amount with respect to non-interesting sequences.

Kits are available for the enrichment of genomic regions of interest with the purpose of sequencing the ROI. These kits are generally known as enrichment kits. The known enrichment kits use the depth of coverage, i.e. the number of independent sequences that read the same position in the sequenced region, as a quality parameter, expressed as the average value on the entire selected region. Generally, for each kit, average ROI coverage values are indicated. This is done by counting the number of reads within target regions of interest and calculating the coverage for each base of the sequence of the region. An important factor in identifying genetic variants in genome analysis is a uniform distribution of reads coverage throughout the ROI. This homogeneity is not easily achievable due to the complexity of the genomes, and the results obtainable with the currently available enrichment kits and sequencing techniques are not completely reliable. To address this problem, in general, the known enrichment kits provide, as a quality parameter, information about the average coverage percentage of the target regions and the length of said regions.

The enrichment kits currently on the market use the depth of coverage as quality parameter.

The inventors realized that with the currently used massive parallel short reads sequencing techniques, some genetic variants may not be called because they are not present in the genome in analysis (i.e., in a genomic sample), or they may not be called because they are not detected even if present in the genomic sample analyzed. In this view, with the currently available enrichment kits and the currently used quality parameters, it is not possible to know if a variant in a certain genomic position captured with currently available kits can be properly identified. For example, the gene SMN1, when mutated, causes spinal muscle atrophy. The gene SMN1 differs from the SMN2 gene for a few nucleotides and is not distinguishable from the latter with the currently available kits, even if well covered by the reads. WO2016/022833A1 discloses a method that aims to quantify a plurality of specific nucleic acid molecules designing a probe set that is sequenced and mapped on a reference to count the number of reads that overlap the target nucleic acid molecules. The examples of WO2016/022833A1 relate to the evaluation of differential expression levels of specific transcripts between two samples, the evaluation of the differential expression levels of specific transcripts between two samples without genome alignment, the evaluation of differential expression of specific transcripts mapping forward reads, the evaluation of the different expression levels of specific transcripts using forward reads, the determination of copy number variation (CNV) by DNA sequencing, the determination of copy number variation (CNV) in cancer cell line by DNA sequencing, and the rapid library generation for determination of copy number variation by DNA sequencing.

US2015/203907A1 discloses a method that aims to replicate the array CGH (comparative genomic hybridization) detection of CNVs (copy number variations) with NGS (new generation sequencing) probes.

US2015/211054A1 discloses a method that uses NGS technology to sequence long fragments of DNA (~50Kb or ~150Kb). Such technology is used to phase haplotypes in target regions. US2015/211054A1 discloses that, ultimately, the workflow provides phased chromosomal sequences as output. US2015/211054A1 defines "phasing" (also referred to as "phased sequencing") as obtaining the sequence and/or alleles associated with one chromosome, or portion thereof, of a diploid or polyploid chromosome.

Ítalo Faria Do Valle et al.: "Optimized pipeline of MuTect and GATK tools to improve the detection of somatic single nucleotide polymorphisms in whole-exome sequencing data", BMC Bioinformatics 2016, vol. 17 (suppl. 12) discloses a method that merges variants called by two algorithms known in the art to improve the calling of somatic variants in which one of the two alleles has a coverage that is much lower than the other allele.

Ferrarini Alberto et al.: "The Use of Non-Variant Sites to Improve the Clinical Assessment of Whole-Genome Sequence Data", PLOS ONE, vol. 10, no. 7, July 2015 (2015-07) discloses a method wherein the genotypability is used to verify a given set of clinical variants using a subset of the gVCF format that was called eVCF. Ferrarini et al. discloses that the eVCF format extends the VCF reporting the genotype of the targeted clinical sites to reduce the amount of data to be analysed in clinical practice.

### Summary of the invention

It is an aim of the present invention to solve the above mentioned problems and to provide a method for the enrichment of selected genomic regions which provides an improved genotypability, i.e., an improved calling of the genotype, of a selected genomic region.

Another aim of the present invention, is to provide a method for the enrichment of selected genomic regions which provides detailed information on genotypable and non-genotypable positions and on non-genotypable variants present therein.

Still another aim of the present invention is to provide a method for the enrichment of selected genomic regions which allows the identification of an effective analysis system (library, e.g. library length, and sequencing techniques) in view of the genomic region to be analyzed.

These and other aims are achieved by the method for the enrichment of genomic regions according to claim 1.

Another object of the invention is an enrichment kit as obtainable with the method of the present invention, according to claim 10.

Preferred embodiments are object of depending claims.

### Brief description of the drawings

- Figure 1 is a schematic representation of 3 possible situations (A, B and C) occurring during the sequencing of fragments having different lengths.
- Figures 2A and 2B schematically shows the alignment of reads obtained when two libraries having different average length of the fragments (i.e., 200 bp in Figure 2A and 350 bp in Figure 2B) are sequenced.
- Figure 3 illustrates the performances of 8 different commercially available enrichment kits with reference to coverage percentage and genotypability.
- Figure 4 shows the percentage of genotypable bases in relationship with 3 average lengths of the fragments of a library.
- Figure 5 shows a comparison between the information provided by VCF format file and gVCF format file.

### Detailed description of the invention

The present invention relates to a method for the enrichment of a selected genomic region, comprising the steps of:
a. Selecting a genomic region to be sequenced;
b. Providing at least one probe set targeting said genomic region;
c. Providing at least a library of nucleic acid fragments, said library comprising at least said selected genomic region, wherein said fragments have a predetermined first average length selected so that at least one flanking region of the selected genomic region is included in one or more fragments and wherein said library comprises fragments including at least a portion of an exon and at least a portion of an intron;
d. enriching at least said genomic region in said library through said at least one probe set, to obtain at least one enriched selected genomic region;
e. Sequencing said at least one enriched selected genomic region using at least a first sequencing technique, to obtain at least a first sequencing data pool of said selected genomic region;
f. Processing said at least first sequencing data pool obtaining at least a first computer readable file including genotypability information of said selected genomic region, wherein said step f. comprises the steps of:
   f.a. aligning the sequence data pool obtained in said step e. to a reference genome sequence;
   f.b. determining genomic coordinates of genotypable portions of said selected genomic region;
   f.c. generating at least a first computer readable file including as genotypability information the genomic coordinates of the genotypable portions of said selected genomic region;
   wherein said first computer-readable file is in gVCF format;
   said method further comprising a step of:
g. Generating at least a second computer readable file including non-genotypability information of said selected genomic region, based on said at least first computer readable file, wherein said second computer-readable file includes as non-genotypability information the genomic coordinates of the non-genotypable portions of said selected genomic region, and wherein said second computer-readable file is in BED format.

It has been observed that the coverage of a genomic region is not directly related to its genotypability. For example, high coverage regions may be non-genotypable, i.e, the genotype of high covered region may be non-callable, in view of the presence of pseudogenes, tandem repeats, duplicated genes, gene families and highly repetitive regions, that cause uncertain read alignment on the genome and thus a low mapping quality of the reads.

Furthermore, currently available enrichment kits have been designed in order to reduce the sequencing of off-target regions i.e. regions flanking the ROI, by capturing short DNA fragments that have more chances of multiple mapping, i.e., a low quality of mapping.

Advantageously, the method of the present invention, allows the production of an enrichment kit that provides for a particularly accurate genotypization, of pre-selected genomic regions of interest, not obtainable with the currently available enrichment kits.

According to an embodiment of the method of the present invention, a plurality of probe sets and/or a plurality of libraries and/or a plurality of sequencing techniques is used to obtain a plurality of first computer readable files, and the method further comprises a step of:
f1. Selecting among said plurality of first computer readable file at least one first computer readable file corresponding to the highest genotypability information.

According to a preferred embodiment, at least a second computer readable file including non-genotypability information of said selected genomic region is generated based on said selected at least one first computer readable file corresponding to the highest genotypability information.

As used herein, the term "genotypability", refers to the capability of a determined genomic region or sequence to be genotyped. In other words, the term "genotypability" refers to the capability to determine the genotype, i.e., the nucleic acid sequence (e.g., DNA sequence) of a determined genomic region.

As used herein "genotypability information", refers to data regarding the number of the positions, i.e., bases, and/or portions of a determined genomic region that can be genotyped.

For example, genotypability information may be expressed as percentage of the position (i.e., bases) that may be genotyped when a particular combination of probe set, library fragments length and sequencing technique is used. For example, when a particular combination of probe set, library fragments length and sequencing technique provides the genotypization of 80 positions in a selected genomic region of interest being 100 bp length, the combination provides a genotypability of 80% of the positions of selected genomic region of interest.

When, for example, more than one combination of probe set, library fragments length and sequencing technique is used, each combination may provide for a different genotypability, e.g., 70%, 80% or 90% of the positions of the same selected genomic region of interest. In this case, the "highest genotypability information" is provided by the combination of probe set, library fragments length and sequencing technique that provides for the calling of the genotype of the 90% of the positions of the selected genomic region.

Accordingly, the term "the highest genotypability information", refers to the data provided by the combination (or the combinations) of probe set, library fragments length and sequencing technique that provides for the highest percentage of the positions of selected genomic region of interest that are genotyped, when a plurality of combinations of probe set, library fragments length and sequencing technique are used. Accordingly, a computer readable file which includes the highest genotypabilty information of a genomic region, may be a computer readable file which is obtained when a determined combination of probe set, library fragments length and sequencing technique provides for the genotype of the highest number of positions in a selected genomic region.

According to embodiments, the probe set may be obtained from one or more enrichment kits known in the art.

According to embodiments, when a plurality of libraries is used, each library has a different average length of the nucleic acid fragments. Methods for the production of nucleic acid fragments libraries are known in the art.

The method of the invention provides for an enrichment kit that, advantageously, allows the user to choose a suitable analysis system, i.e. a suitable library and a suitable sequencing platform or sequencing technique, to be used, on the basis of the region of interest to be studied, as well as the type of analysis that has to be performed.

Also advantageously, the method of the invention provides for an enrichment kit that allows the user to know whether a given genetic variant, which is not detected in the sequencing analysis, is actually not present in the sequenced nucleic acid sample or it may not be detected because of, for example, a technical limitation of a specific sequence analysis system.

According to the method of the invention step f comprises the steps of:
f.a. Aligning the sequence data pool obtained in step e. to a reference genome sequence;
f.b. Determining genomic coordinates of genotypable portions of said selected genomic region,
f.c. generating at least a first computer readable file including as genotypability information the genomic coordinates of the genotypable portions of said selected genomic region of said selected genomic region.

According to embodiments, the method further comprises a step of comparing the genomic coordinates of said selected genomic region with the genomic coordinates of said genotypable portions to obtain the genomic coordinates of said non-genotypable portions.

Advantageously, the method of the invention allows to integrate the coverage quality parameters, that may be obtained by processing alignment sequencing data obtained in step f.a. with information on genotypable and/or non-genotypable portions or positions in a genomic region of interest, and/or, with information concerning genetic variants of the region of interest, e.g., the variants recorded in public or internal databases.

As above mentioned, the present invention relates to a method for the enrichment of selected genomic regions, in particular, with the purpose of targeted resequencing of selected genomic regions of interest.

Generally, targeted resequencing is performed by generating a library of nucleic acid fragments deriving from the genomic sample to be analyzed. A targeted enrichment of the fragments which comprise in the region of interest is performed to make the specific region of interest of the genome selectively over-represented in the nucleic acid sample. The enriched selected genomic region of interest is sequenced, for example, using a selected sequencing technique. Several sequencing techniques are known in the art, and may be performed, for example, by a sequencing platform. For example, a sequencing platform may be a device that performs sequencing in an automatic manner, for example according to monodirectional or bidirectional (paired end technology) sequencing.

As used herein, the term "library", refers to a collection of nucleic acid fragments deriving from one or more nucleic acid samples to be sequenced. The average length of the fragments can be pre-selected on the basis of the analysis to be carried out and on the basis of the platform and technique selected to perform the sequencing. Different techniques for the production of sequencing libraries are known in the art. For example, different techniques may allow the production of nucleic acid fragments having different average length, according to the used technique. For example, libraries may be produced by mechanical shearing (e.g., by sonication) of nucleic acid, or by enzymatic shearing, for example, using transposase, for a tagmentation-based library construction. "Tagmentation" is a method for library construction that is known in the art.

Generally, a library is sequenced after the enrichment of a genomic region of interest in the library.

Currently available enrichment kits are designed to provide for the enrichment of libraries having an average length of the fragments selected in order to limit the so called "off target" sequencing, i.e., in order to have a substantially negligible sequencing of flanking regions.

As used herein, the term "off-target sequencing" relates to the sequencing of portion of the genome different from the selected genomic region of interest such as, for example, flanking regions of the genomic regions of interest.

In order to limit the so called "off target" sequencing, it is known in the art to use libraries having fragments as short as possible, to reduce the sequencing of positions outside the genomic region of interest.

According to a preferred embodiment, the method of the invention comprises a step of increasing the average length of the fragments, from the first average length, to increase the genotypability information of said selected genomic region, e.g., to increase the number of the positions of the selected genomic region that are genotyped.

When a fragment of a library comprises a genomic region of interest and one or more flanking regions, both the genomic region of interest and the one or more flanking regions are enriched.

As used herein, the terms "portion", may refer to a single "position" or "base" in a genomic region or to a sequence comprising a plurality of bases.

In order to sequencing a library of nucleic acid fragments, said library comprising at least one selected genomic region, it is possible to sequence at least part of at least one of the sequences flanking said selected region of interest.

It has been observed that, when the length of the library fragments is selected so that at least one flanking region of the selected genomic region is included in the fragment, a more effective mapping of said selected genomic region, with respect to fragments that do not comprise flanking regions, is obtained.

In other words, it has been observed that by increasing the average length of the fragments of a library, an increase in the mappability of the reads obtained through sequencing of the selected genomic region may be obtained. In other words, by increasing the average length of the fragments of a library, an increase in the mappability of the selected genomic region, and thus an increase of the genotypability of the selected genomic region, may be obtained.

For example, it has been observed that an exome that is sequenced by using a library wherein nucleic acid fragments have an average length of about 300 bp, with paired end sequencing at 150x2 (i.e., forming two mates of 150 bp) has 200.000 genotyped positions more than the same exome sequenced in the same way, but using a library wherein nucleic acid fragments have an average length of about 220 bp, at the same coverage. Also, when for example paired end sequencing at 150x2 is performed using a library of fragments having average length of 220 bp, reads that are generated overlap 80 bp. In other words, for each fragment of 220 bp, 80 bp of the fragments are sequenced two times, without providing any advantage in the analysis.

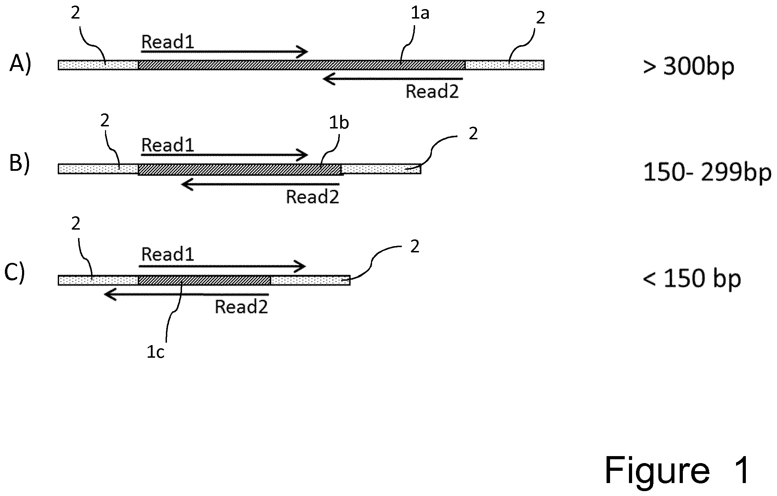

As illustrative, non-limiting example, Figure 1 shows a schematic representation of 3 possible situations (A, B and C) occurring during the sequencing of fragments having different lengths. In particular, Figure 1 schematically represents a 150x2 paired-end sequencing of 3 fragments of nucleic acid 1a, 1b and 1c, having different length, namely >300 bp (case "A"), between 150 and 299 bp (case "B") and <150 bp (case "C"). Each fragment is contained between 5' and 3' adapter oligonucleotides 2, that do not relate to the genomic nucleic acid to be sequenced. The indicated length >300 bp (case "A"), between 150 and 299 bp (case "B") and <150 bp (case "C"), refer to the length of the fragments of genomic nucleic acid, and do not include the adapter oligonucleotides 2.

When paired-end sequencing is carried out, both ends of the nucleic acid (e.g., DNA) fragments in a library are sequenced. In this case, 2 reads ("Read 1" and "Read 2", according to Figure 1) each one having a length of 150 bp are produced for each fragment.

In cases "B" and "C" of Figure 1, since the sequencing technology selected (150x2 paired-end sequencing) produces reads of 150 bp, part of the fragments 1b and 1c is sequenced two times. In other words, at least part of the two reads "Read1" and "Read2", that are at least partially overlapping, provide for the sequencing of the same portion of the fragments 1b, 1c. In particular, in case "C", where the fragment have a length that is less than 150 bp, the two reads provide for the sequencing of part of the two adapter oligonucleotides 2, which do not relate to the genomic nucleic acid to be sequenced.

Conversely, when the fragment is more than 300 bp length (case "A") Read1 and Read2 do not overlap, so that the double sequencing of the same portion of the fragment 1a is avoided.

Also, advantageously, by increasing the length of the fragments, the probability that a fragment includes at least a flanking region of the genomic region of interest increases. For example, when an exome is taken as genomic region of interest, the length of fragments of the library is preferably selected to be more than the average length of exons. This would increase the possibility to obtain fragments comprising at least an exon, i.e., a portion of the region of interest to be sequenced, and at least one flanking region, i.e., an intronic sequence. It has been observed that, when sequencing is carried out using a library comprising fragments including at least a portion of an exon, which is, in turn a portion of the exome, and at least a portion of an intron, an improving in the mappability of the reads is obtained. A possible explanation is that introns are more polymorphic compared to exons. Thus, the association of the sequencing data related to an intronic portion with data related to an exonic portion increases the quality of the mapping of the exonic portion of the sequenced fragment.

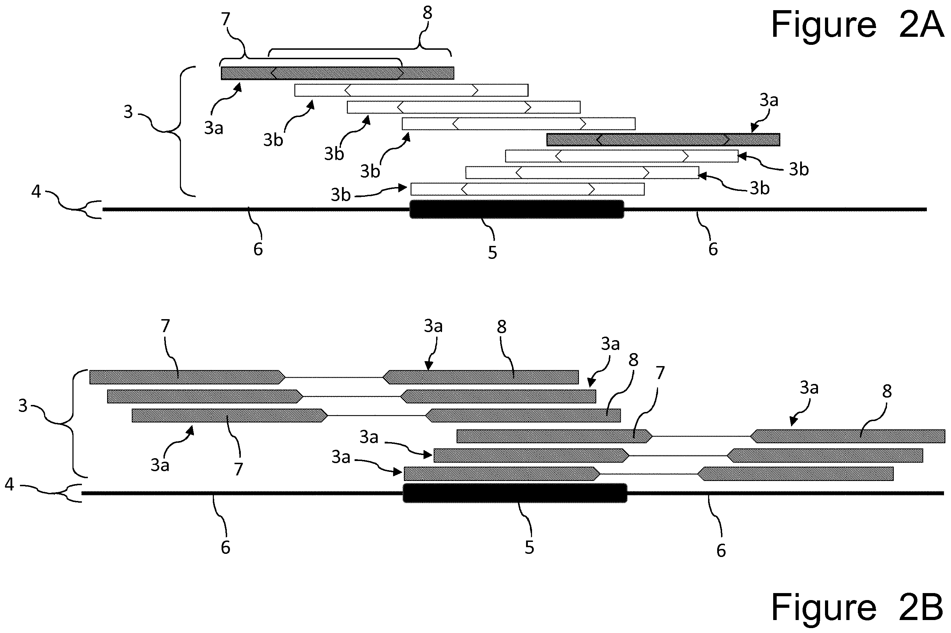

Figures 2A and 2B schematically represent the alignment of the reads obtained by sequencing, in this case with a paired-end sequencing technique, of two libraries, each one having a different average fragment length.

In particular, Figures 2A and 2B show the results obtained by enriching, using the same probe set, the same region of interest in two libraries having different average fragments' length, and then sequencing it using the same sequencing technique.

In particular, Figure 2A refers to the sequencing of a library having average fragments' length of 200 bp, and Figure 2B refers to the sequencing of a library having average fragments' length of 350 bp.

Figures 2A and 2B show a plurality of paired reads 3, each one comprising a left mate 7 and a right mate 8, i.e., a left read and a right read, obtained from the sequencing (i.e., paired-end sequencing) of fragments of two libraries (a first library having average fragments' length of 200 bp in Figure 2A, and a second library having average fragments' length of 350 bp in Figure 2B). In Figures 2A and 2B a plurality of paired reads 3 are aligned to a reference genomic sequence 4.

With the alignment of the reads obtained with sequencing, it is possible to evaluate the mapping quality of the reads.

In Figure 2A and 2B, high-quality mapping paired reads 3a and low-quality mapping paired reads 3b are shown.

In Figures 2A and 2B, the reference genomic sequence 4 comprises a genomic region of interest 5 (ROI) flanked by two flanking regions 6. In other words, the reference genomic sequence 4 comprises a pre-selected genomic region to be sequenced, namely, a target genomic region (i.e., genomic region of interest 5 ), between two flanking regions 6, namely, two off-target genomic regions (i.e., regions outside the selected genomic region of interest).

For example, in Figures 2A and 2B, the genomic region of interest 5 may be an exon and the two flanking regions 6 may be introns.

Figure 2A, which is merely illustrative and non-limiting, schematically shows that when a library having average fragments' length of 200 bp is used, a majority of low-quality mapping paired reads 3b, with respect to high-quality mapping paired reads 3a, may be obtained. Conversely, Figure 2B, which is merely illustrative and non-limiting, schematically shows that when a library having an increased average fragments' length, e.g. 350 bp, is used, the presence of low-quality mapping paired reads 3b may be reduced.

Figure 2A and 2B are to be intended as being merely illustrative and not limiting of the scope of the invention. For example, low-quality mapping paired reads 3b may be obtained also when a library of 350 bp is used. However, it has been observed that, when the average length of the fragments of a library increases, the number of high-quality mapping paired reads 3a increases.

With reference to the exemplary case of Figure 2B, the high-quality mapping paired reads 3a have one of the two mates, i.e., one of left mate 7 and right mate 8, that maps univocally in one of the two flanking regions 6, e.g., in the intronic region and, transfers the high quality alignment to the corresponding right mate 8 of left mate 7. Advantageously, a position of the region of interest 5 that, for example, is not genotyped when a library having average fragments' length that provides for a low quality mapping (as shown, for example, in Figure 2A) may be genotyped by using a library having longer fragments (as shown, for example, in Figure 2B).

A sequencing library may be prepared by fragmentation of a DNA sample, followed by 5' and 3' adapter ligation.

For example, adapters may be oligonucleotides having a predetermined sequence that may be bound to the 5' and 3' end of each fragment in a sequencing library. For example, the oligonucleotides may be used to immobilize the fragments onto a support for the sequencing reaction.

According to the present description, when reference is made to the length of the fragments a library, it is indicated the length of the fragment deriving from the genomic nucleic acid of a sample, excluding adapters and oligonucleotides that may be bound to the 5' and 3' end of each genomic fragment during the production of the library. According to the present description, one or more fragment of the library may include at least a portion of the selected genomic region of interest and one or more flanking region of said portion.

According to an aspect, the kit of the present invention does not include a library of nucleic acid fragments.

As used herein, the terms "platform" and "sequencing platform" refer to an instrument suitable for nucleic acid sequencing. Sequencing platforms are known in the art, and different platforms may use different sequencing techniques.

As used herein, the terms "sequencing technique" and "sequencing techniques" refer to methods that are used for determining the sequence of a strand of nucleic acid, e.g., DNA. Several sequencing techniques are known in the art. An exemplary sequencing technology is paired ends sequencing. For example, different platforms may provide for reads having different average length.

As used herein, the terms "enrichment" and "targeted enrichment" refer to a procedure wherein specific sequences (for example, specific regions of interest of a genome) are selectively made to be over-represented in a nucleic acid (e.g. DNA) sample. Techniques for targeted enrichment are known in the art; for example, such techniques may be based on PCR or capture through hybridization.

As used herein, the terms "coverage" and "depth of coverage" refer to the number of reads that include a given nucleotide in a given position with respect to a reference sequence that is being re-sequenced; i.e., the number of nucleotides from reads that are mapped to a given position.

As used herein, the terms "read", "reads" and "sequencing length/s", refer to nucleotide sequence information that is obtained from a sequencing reaction. In other words, these terms refers to the information contained in a piece of nucleic acid that is sequenced ("read") by the sequencers, i.e., by the sequencing platform. When a paired end sequencing is carried out, the two "reads" generated for the same fragment of nucleic acid are known also as "mates".

As used herein, the term "call", "calling", and "variant call", refer to a phase of the bioinformatic analysis of data obtained with an alignment, wherein the variants of a sequence in a region of interest with respect to a reference genome are identified. In this view, a "non-callable variant" is a variant that cannot be identified. As used herein, the term "genotypable" is a synonym of the term "callable".

As used herein, the term "enrichment kit" refers to a set of tools (comprising, e.g., probes, reagents, primers) to carry out an enrichment procedure, as defined above. In particular, the probes are selected to target and capture a selected genomic sequence of interest.

According to embodiments, the enrichment kit may comprise the reagents for the preparation of a sequencing library.

The genomic regions of interest (ROIs) are, for example, small sets of genes or small genomic regions that are potentially implicated in complex diseases; such genomic regions may be selected on the basis of experimental data derived from, for example, genome-wide association studies (GWAS), pharmacogenetics and pathway analysis experiments.

The at least one probe set provided in step b. of the method of the invention may be a known probe set.

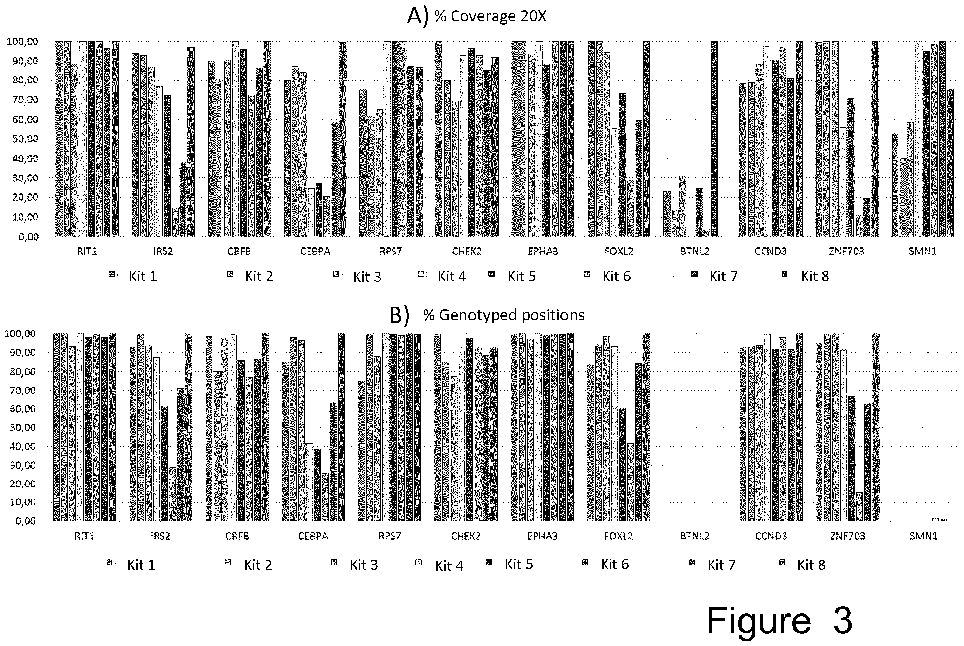

It has been observed that the coverage of a genomic region is not directly related to its genotypability, and that an enrichment kit may provide for a good coverage of a genomic region of interest, but for a low mapping quality and low genotypization. Figure 3 illustrates the performances of 8 different commercially available enrichment kits (Kit 1 - Kit 8) with reference to coverage percentage and genotypability. Data related to 12 exemplary genes (RIT1, IRS2, CBFB, CEBPA, RPS7, CHEK2, EPHA3, FOXL2, BTNL2, CCND3, ZNF703 and SMN1) were collected and analyzed. Known kits comprise known probe sets for one or more genes.

Sequencing data were obtained by using a library fragment length of 200-220 bp and the same sequencing technique. Average sequencing coverage was 80X.

Panel "A" of Figure 3 shows results concerning coverage performances, indicated as percentage of positions (i.e., bases) that are covered at 20X (i.e., bases that have been mapped in the same position by 20 different reads).

Panel "B" of Figure 3 shows results concerning genotypization performances, indicated as percentage of position (i.e., bases) that are genotyped when a selected enrichment kit is used.

Figure 3, shows that a kit that provides for a good coverage of a genomic region using a predetermined library fragments' length and using the same sequencing technology, may not provide for a good genotypability of the same region.

For example, Kit 7 provides for a high coverage of genes BTNL2 and SMN1 (around 100% coverage 20X), but do not allow the genotypization of the same genes. Conversely, for example, Kit 1 and Kit 2 provides for a coverage of gene FOXL2 that is higher than the coverage provided by Kit 3, but Kit 3 allows a better genotypization of gene FOXL2, with respect to Kit 1 and Kit 2.

In step f. of the method of the invention at least a first computer readable file including genotypability information of the selected genomic region is obtained. For example, data on genotypability of a selected genomic region may be indicated as percentage of genotyped positions (i.e., bases) with respect to the total number of position of the selected genomic region.

According to the invention, the at least one first computer readable file includes, as genotypability information, the genomic coordinates of the genotypable portions of said selected genomic region.

Advantageously, the method of the present invention allows the user to select the combination of probe set, library length and sequencing technique that provides for the highest genotypability of the region of interest.

As above mentioned, it has been observed that, by increasing the average length of the fragments of a library, an increase in the genotypability of the selected genomic region, may be obtained.

For example, Table 1, here below reported, shows the percentage of genotypable bases in different genes of interest.

The results reported in Table 1 were obtained by using the same probe set and the same sequencing technique for each gene, in combination with three libraries having different average fragments' lengths, namely 200 bp, 350 bp and 500 bp.

The line entitled "All" refers to the results obtained for all the analyzed genes, considering the sum of the length of all the genes and the total percentage of genotypable bases on the total of the bases of the genes.

A selected genomic region according to the invention may comprise a plurality of genes.

| **Table 1** | | | | |
|---|---|---|---|---|
| | | **% of genotypable bases** | | |
| **Genes of interest** | **Length (bp)** | **200** | **350** | **500** |
| **All** | 41356 | 97,66 | 98,93 | 99,53 |
| **ABCA4** | 6822 | 96,20 | 99,30 | 99,60 |
| **AGBL5** | 2726 | 98,20 | 98,40 | 99,00 |
| **AIPL1** | 1184 | 95,00 | 100,00 | 100,00 |
| **ARHGEF18** | 3522 | 100,00 | 100,00 | 100,00 |
| **ARL2BP** | 492 | 98,30 | 99,10 | 100,00 |
| **ARL6** | 561 | 97,00 | 98,10 | 98,90 |
| **BBS2** | 2166 | 98,10 | 99,10 | 100,00 |
| **BEST1** | 2014 | 98,60 | 99,00 | 100,00 |
| **C2orf71** | 3867 | 96,10 | 98,20 | 99,30 |
| **C8orf37** | 624 | 97,00 | 100,00 | 100,00 |
| **CA4** | 939 | 100,00 | 100,00 | 100,00 |
| **CDH23** | 10489 | 94,80 | 96,00 | 98,80 |
| **CDHR1** | 2778 | 98,30 | 99,50 | 99,80 |
| **CERKL** | 1677 | 100,00 | 100,00 | 100,00 |
| **CIB2** | 564 | 100,00 | 100,00 | 100,00 |
| **CLRN1** | 931 | 95,00 | 96,20 | 98,00 |

As can be observed from Table 1, the percentage of the genotypable bases increases with the increasing of the average length of the fragments of the library.

Of course, when the 100% of the positions is genotyped at a determined length of the fragments, the increase of the length of the fragments may be stopped.

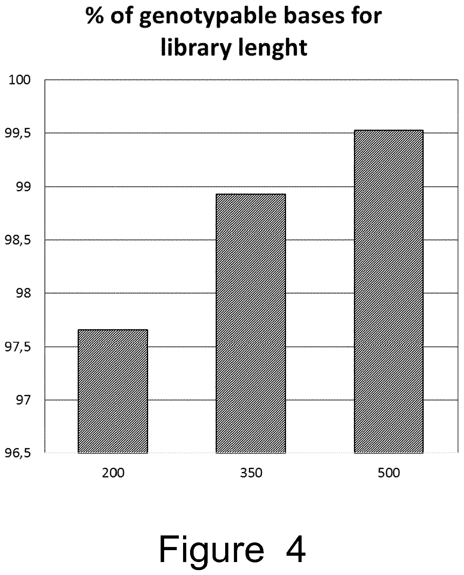

Figure 4 schematically shows the results obtained with reference to all the genes reported in Table 1, i.e., Figure 4 shows the results obtained with reference to the line "All" of Table 1.

Figure 4 shows that, when all the genes in Table 1 are selected as genomic region of interest, an increase in the percentage of the genotypable bases of the genomic region may be obtained by increasing the average length of the fragments of the library, when using the same probe set and the same sequencing technology.

According to a preferred embodiment, the method of the invention comprises a step of increasing the average length of the fragments, from the first average length, to increase the genotypability information of said selected genomic region, e.g., to increase the number of the positions of the selected genomic region that are genotyped.

According to embodiments of the method of the invention, in step c. of the method a plurality of libraries of nucleic acid fragments are provided, wherein each one of said libraries have a different predetermined average length of the fragments, and said step e. is carried out with a plurality of different sequencing techniques, to obtain a plurality of sequencing data pools for each combination of library and sequencing technique.

A plurality of probe sets is used to enrich a selected genomic region in a plurality of libraries, each library having a different fragments' length, to obtain a plurality of enriched selected genomic regions.

Said enriched selected genomic regions are sequenced using a plurality of sequencing techniques, to obtain a plurality of sequencing data pools related to the selected genomic region.

Said plurality of sequencing data pools are processed to obtain a plurality of first computer readable files including genotypability information of the genomic region of interest, for example, including the genomic coordinates of the genotypable portions of the genomic region.

According to the invention, each first computer readable file includes genotypability information of the genomic region of interest, based on a determined combination of probe set used, library fragment's average length used and sequencing technique used. According to embodiments, when a plurality of first computer readable files including genotypability information of the genomic region of interest is obtained, at least one first computer readable file providing the highest genotypability information is selected among said plurality of first computer readable files. According to embodiments, said at least one first computer readable file providing the highest genotypability information is included in the kit of the invention.

It has been observed that different combination of probe set, library fragment's average length and sequencing technique provides for different genotypability information.

According to the invention at least a second computer readable file including non-genotypability information of said selected genomic region is generated based on the selected first computer readable file.

According to the invention, the second computer-readable file includes as non-genotypability information the genomic coordinates of the non-genotypable portions of said selected genomic region, and it is in BED format.

Optionally, an enrichment kit may comprise reagents for the preparation of a library from a genomic nucleic acid sample, and for the targeted enrichment of selected genomic regions of interest.

According to an aspect, the kit of the present invention does not comprise a library of nucleic acid fragments.

For example, an enrichment kit may be based on PCR, hybridization probes, amplicons, or other methods of enrichment.

Examples of enrichment kits known in the art are: Qiagen QIAseq Targeted DNA Panels, Illumina Nextera^{®} Custom Enrichment Kit, Agilent SureSelect Target Enrichment System, Illumina TruSeq ^{®} Custom Enrichment Kit, Roche Nimblegen SeqCap EZ Target Enrichment Kit, and NEB (NebNext Direct).

The at least one probe set, and optionally the reagents comprised in the enrichment kit, may be produced *ad hoc*, for example, on the basis of the regions of interest and of the type of analysis to be carried out. Methods for the production of probe sets and reagents thereof are known in the art.

After the regions of interest have been selected and at least one suitable probe set has been provided, the probe set is used to enrich the selected genomic region in a plurality of libraries of nucleic acid fragments, wherein each library has a different average fragments length, and the obtained plurality enriched selected genomic regions (each enriched genomic region deriving from a determined library) are sequenced using a plurality of sequencing techniques (for example, different sequencing techniques may provide for reads having different, predetermined, average length), in order to identify the non-genotypable portions of the ROI when a determined combination of probe sets, library fragments' length and sequencing technique used. In other words, the probe sets may be tested with different sequencing systems, i.e., different combinations of libraries (preferably each one having a different average length of the nucleic acid fragments) and sequencing techniques. In this way, the regions that are genotypable (i.e., callable) and not genotypable with the selected probe set, when used in combination with a specific combination of libraries and sequencing technique, can be identified.

According the invention, in step c. a plurality of libraries of nucleic acid fragments are provided, for example produced, wherein the fragments of each library have a different predetermined average length (for example, 220 bp, 350 bp and 500 bp), and said step e. is carried out with a plurality of different sequencing techniques, to obtain a plurality of sequencing data pools, wherein each sequencing data pool relates to a determined combination of library and sequencing technique (for example, 2x75, 2x150 and 2x250 paired end sequencing, or 200 bp, 500 bp and 800 bp single reads).

At least one sample of genomic nucleic acid is used to provide, e.g., produce, a library comprising nucleic acid fragments having a determined average length.

A plurality of sequencing libraries, each one comprising fragments having a different average length are produced.

The enrichment of the selected region of interest is then carried out on the library (or the different libraries) with a pre-selected probe set.

Subsequently, at least part of the enriched selected genomic regions deriving from the library obtained from the nucleic acid sample is sequenced.

Each enriched genomic region is then sequenced to obtain a sequencing data pool concerning the genomic region itself.

Different sequencing techniques may be used for the sequencing of the ROI (region of interest).

A plurality of nucleic acid samples is used to obtain a plurality of libraries. The fragments of different libraries generated from different samples may have the same average length or a different average length.

The selected genomic region of interest is enriched by means of at least one probe set in all the libraries. When a fragment of a library comprises a genomic region of interest and one or more flanking regions, both the genomic region of interest and the one or more flanking regions are enriched. Then the enriched regions of interest, i.e., the same genomic region that has been enriched in different libraries deriving from the same or from different samples, may be sequenced. Preferably, all the different libraries are sequenced with more than one sequencing technique. In other words, the probe set targeting the genomic region of interest is preferably tested with different combination of average length of the fragments in the libraries and sequencing techniques, to obtain a plurality of first computer readable files including genotypability information for each combination.

As above mentioned, according to embodiments, a plurality of sequencing libraries, each one comprising fragments having a different average length are produced. According to embodiments, each library is sequenced with a plurality of different sequencing techniques, e.g. one or more sequencing technologies that provide for reads having different average length.

The at least first sequencing data pool obtained in step e. of the method of the invention is processed with bioinformatic tools.

For each combination of length of library and sequencing techniques, a sequencing data pool is obtained and processed to provide genotypability information of the selected region of interest and, optionally, on variants callability.

Advantageously, the method of the present invention allows the user to select the combination of library length and sequencing technique that provides for the highest genotypability of the region of interest, For example, by selecting among a plurality of first computer readable files including genotypability information of the region of interest, the first computer readable file which corresponds to the combination of probe set, library fragments' length and sequencing technique which provides for the highest genotypability of the region of interest.

Advantageously, the genotypability of a selected ROI may be evaluated by testing a plurality of combination of probe sets/libraries/sequencing techniques, so that an enrichment kit can be provided with probes, reagents and information selected to provide a library wherein the fragments have an average length that provide for an effective genotyping when used in combination with a predetermined sequencing technique.

The sequencing data pool (or the plurality of sequencing data pools) obtained in step e. of the method of the invention, are aligned to a reference genome (for example, the human reference genome GRCh37 or GRCh38, available from the Genome Reference Consortium) using a sequence alignment software. Suitable bioinformatic software tools for alignment are known in the art, such as BWA, Isaac, etc.

The results of the alignment of the at least one sequencing data pool with the sequence of the reference genome may be collected in a computer readable file, preferably in BAM format.

The BAM format (.bam) is a known file format. In the BAM format is the compressed binary version of the Sequence Alignment/Map (SAM) format, a compact and indexable representation of nucleotide sequence alignments.

The BAM files may be subsequently used to determine the genotype of each position of the region of interest, and to generate a computer readable file including the genomic coordinates of genotypable bases or portions in the ROI.

The BAM file is processed to generate a first computer readable file including as genotypability information the genomic coordinates of the genotypable portions of the selected genomic region.

The first computer readable file containing, i.e., including, genotypability information, the genomic coordinates of the genotypable portions of the selected genomic region, in the ROI is in gVCF format. Software that convert BAM files into gVCF files are known in the art; exemplary software are GATK (Broad) and Isaac (Illumina).

The gVCF (genome Variant Call Format) is a known file format. gVCF is a set of conventions applied to the standard "variant call format" (VCF). These conventions allow representation of genotype, annotation and other information across all sites in the genome (or in a region of interest of the genome) in a reasonably compact format. However, gVCF format file is generally not used in currently available sequencing methods and it is not supported by many variant analysis and interpretation softwares.

Advantageously, in addition to the genomic coordinates of the genotypable portions of the selected genomic region, gVCF file also reports the genotype of invariant (reference) positions.

In particular, it has been observed that the VCF file, which is a known standard format file to analyze variants in a selected genomic sequence, provides the variants that can be called by processing the alignment data (e.g., by processing the data included in a BAM file) but does not provide information on the genotypability of the selected genomic region. For example, the lack of a variant in the VCF can be due to the invariant position or to the lack of genotypability of that position Conversely, as above mentioned, in addition to the genomic coordinates of the genotypable portions of the selected genomic region, gVCF file also reports the genotype of invariant (reference) positions.

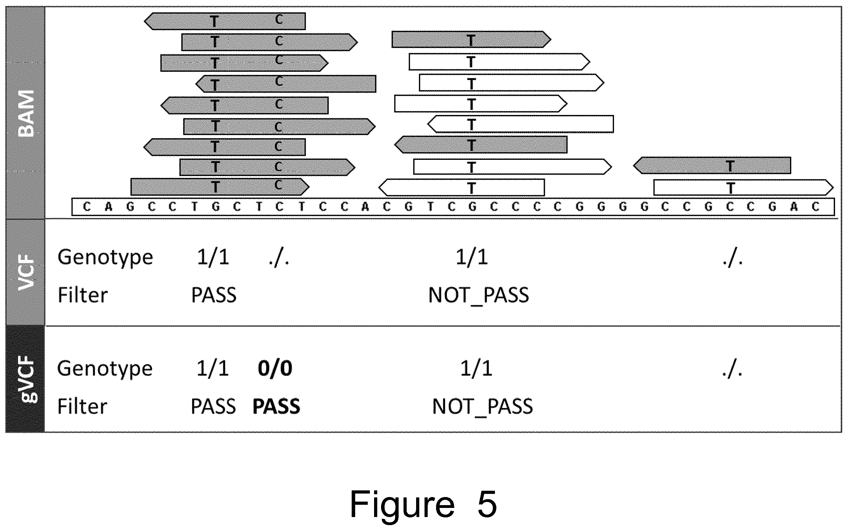

For example, Figure 5 show a comparison between the information provided by VCF format file and gVCF format file. As can be observed, by processing the same alignment data, reported in a BAM file, to obtain a gVCF file it is possible to obtain information on callability of position that may not be obtained by converting the BAM files into VCF files.

In particular, by converting BAM files in gVCF files it is possible to apply filters that provide for the identification of position that are well covered but do not present variants with respect to the reference genome (indicated as "0/0 PASS" in Figure 5). This is not possible with VCF files, wherein positions that are well covered but do not present variants with respect to the reference genome are indicated in the same way of position that are not sufficiently covered (i.e., "./." in Figure 5).

Both VCF and gVCF indicate as "1/1 NOT_PASS" those positions that are sufficiently covered, but wherein a variant cannot be called because of a low quality of alignment.

According to the invention, the first computer readable file containing genotypability information, i.e.the genomic coordinates of the genotypable portions of the selected genomic region is in gVCF format.

According to invention, a second computer readable file including non-genotypability information of said selected genomic region, based on the first computer readable file (gVCF) is generated.

The gVCF file may be subsequently used to generate a second computer readable file including non-genotypability information of the selected genomic region; non-genotypability information are the genomic coordinates of the non-genotypable portions of the genomic region of interest. The computer readable file including the genomic coordinates of the non-genotypable positions in the ROI, is in BED format. The BED (Browser Extensible Data, .bed) format is a known file format, providing a flexible way to define data lines that are displayed in an annotation track. The Applicant developed a proprietary software that converts gVCF files into BED files. The BED file can be uploaded into suitable software for sequencing data analysis, in order to provide information to the user concerning the portion and/or positions of the region in analysis that are non-callable (i.e., non-genotypable) with the combination of library fragments' length and sequencing technique selected to carry out the analysis.

According to the present invention, the BED file includes a listing of the non-genotypable portions of the genomic region of interest, and the genomic coordinates of these non-genotypable portions.

The second computer readable file obtained in step g. of the method of the invention, including information on non-genotypable positions of the genomic region of interest, the genomic coordinates of non-genotypable positions of the genomic region of interest, is in BED format, and it is included in the enrichment kit.

The step of comparing the genomic coordinates of the selected genomic region with the genomic coordinates of the genotypable portions to obtain the genomic coordinates of the non-genotypable portions comprises a step of subtracting the genomic coordinates of the genotypable portions from the genomic coordinates of the genomic region.

The genomic coordinates of the non-genotypable portions and positions may be obtained by subtracting the genomic coordinates of the genotypable portions and positions (listed in a gVCF computer readable file), from the genomic coordinates of the region of interest.

According to embodiments, the method of the invention further comprises step h. of producing a list of non-genotypable variants, preferably clinically-relevant variants, of said genomic region. Preferably, said variants are preselected variants. In other words, the kit of the present invention may further comprise a list of variants that are not genotypable with the kit, when the kit is used with a particular combination of library and sequencing read length.

The list of non-genotypable variants may be obtained by processing a gVCF file. Clinically relevant variants, are genetic variants of the region of interest possessing medical relevance, for example, that may cause a disease or may be involved in a disease.

The computer readable file including the above mentioned list of preselected variants that are not genotypable is provided in XLS or PDF format. The present invention also relates to a kit for the enrichment of a selected genomic region in a genomic nucleic acid sample, as obtainable with a method according to the invention.

A kit for the enrichment of a selected genomic region in a genomic nucleic acid sample as obtainable with a method according to the invention, comprises at least one probe set targeting said selected genomic region, at least one first computer readable file including genotypability information of said selected genomic region and at least one second computer-readable file including non genotypability information of said selected genomic region and/or at least a list of non-genotypable variants of said genomic region, wherein said genotypability information, said non-genotypability information and said a list of non-genotypable variants of said genomic region are based on the use of said at least one probe set in combination with a library having a predetermined fragments' length selected so that at least one flanking region of the selected genomic region is included in one or more fragments and wherein said library comprises fragments including at least a portion of an exon and at least a portion of an intron, and with the use of a predetermined sequencing technique, wherein said at least one first computer-readable file is in gVCF format and includes the genomic coordinates of the genotypable portions of said genomic region and wherein said at least one second computer-readable file is in BED format includes the genomic coordinates of non-genotypable portions of said genomic region.

According to the invention, said at least one first computer-readable file includes the genomic coordinates of the genotypable portions of said genomic region, and is in gVCF format.

According to the invention, the second computer-readable file includes the genomic coordinates of the portions that cannot be genotyped, i.e. that are non-genotypable, in said genomic region when said kit is used, and is in BED format.

According to embodiments, said at least one first computer-readable file includes the highest genotypability information.

According to embodiments, said second computer-readable file is based on the at least one first computer-readable file including the highest genotypability information.

According to embodiments, the kit may comprise a listing of the non-genotypable variants, preferably in XLS or PDF format, of the selected genomic region. According to the invention, the kit comprises at least one first computer readable file including genotypability information of the selected genomic region.

According to embodiments, the kit may further comprise reagents for the preparation of at least one sequencing library.

According to the invention, the kit comprises at least a probe set useful for target enrichment of a selected genomic region in a library of nucleic acid fragments, and a second computer readable file including the list of the non-genotypable positions of the region of interest and the genomic coordinates thereof, in BED format. According to embodiments, the kit may include reagents for the preparation of one or more libraries having a predetermined average fragment length.

Preferably, average fragment length of the libraries are selected on the basis of the sequencing technique to be used.

As above mentioned, advantageously, the first computer readable file including genotypability information on the genotypable positions of the region of interest and/or the second computer readable file including information on the non-genotypable positions of the region of interest and the genomic coordinates thereof is included in the enrichment kit, and provides support to operators in choosing an optimum length of library and sequencing technique to be used.

The enrichment kit of the invention, which may be based for example on hybridization probes, amplicons or other methods of enrichment, comprises at least one computer readable BED file, wherein non-genotypable portions or positions of the ROIs are listed, in particular with reference to a specific sequencing systems (i.e., combination of libraries and sequencing technologies). Advantageously, the BED file allows to discriminate whether a genetic position is genotypable (i.e., callable) in a sequenced nucleic acid sample or not, regardless to the coverage of said position. According to embodiments, the kit of the present invention may further comprise a list of preselected variants that are not genotypable with the kit, when the kit is used with a particular combination of library and sequencing read length.

Preselected variants may be, for example clinically relevant variants, i.e., genetic variants of the region of interest possessing medical relevance; namely, that may cause a disease or may be involved in a disease.

Preferably, the computer readable file including the above mentioned list of preselected variants that are not genotypable is provided in XLS or PDF format.

The enrichment kits obtained with the method of the invention are suitable for targeted resequencing of specific genomic regions of interest (ROI).

Advantageously, the method of the invention allows the production of enrichment kits that include detailed information on the genotypability of the ROIs, in particular, on the portions of the region of interest that are genotypable and/or not genotypable when the kit is used in combination with a determined library and sequencing platform (sequencing technique). According to the invention, these information are included in computer readable files: at least a first computer readable file including information on genotypable portions, preferably the genomic coordinates of genotypable portions, of a selected genomic region, and/or at least a second computer readable including information on non-genotypable portions of a selected genomic region of interest, preferably the genomic coordinates of non-genotypable portions and/or positions of the region of interest. In particular, it has been observed that the effective genotypability of the ROIs is correlated with the length of the selected library and with the selected sequencing technique used (i.e., platforms).

Advantageously, the information concerning genotypable and/or non-genotypable portions and/or positions of the region of interest, as included in the enrichment kits of the present invention, provides support to operators in choosing optimum length of library and sequencing technique to be used, on the basis of the selected enrichment kit and of the genomic regions of interest.

The kit of the present invention is advantageously suitable for industrial application, scientific research and clinical application.

## Claims

1. A method for the enrichment of a selected genomic region, comprising the steps of:
a. Selecting a genomic region to be sequenced;
b. Providing at least one probe set targeting said genomic region;
c. Providing at least a library of nucleic acid fragments, said library comprising at least said selected genomic region, wherein said fragments have a predetermined first average length selected so that at least one flanking region of the selected genomic region is included in one or more fragments and wherein said library comprises fragments including at least a portion of an exon and at least a portion of an intron;
d. enriching at least said genomic region in said library through said at least one probe set, to obtain at least one enriched selected genomic region;
e. Sequencing said at least one enriched selected genomic region using at least a first sequencing technique, to obtain at least a first sequencing data pool of said selected genomic region;
f. Processing said at least first sequencing data pool obtaining at least a first computer readable file including genotypability information of said selected genomic region, wherein said step f. comprises the steps of:
f.a. aligning the sequence data pool obtained in said step e. to a reference genome sequence;
f.b. determining genomic coordinates of genotypable portions of said selected genomic region;
f.c. generating at least a first computer readable file including as genotypability information the genomic coordinates of the genotypable portions of said selected genomic region;
wherein said first computer-readable file is in gVCF format;
said method further comprising a step of:
g. Generating at least a second computer readable file including non-genotypability information of said selected genomic region, based on said at least first computer readable file, wherein said second computer-readable file includes as non-genotypability information the genomic coordinates of the non-genotypable portions of said selected genomic region, and wherein said second computer-readable file is in BED format.

2. Method according to claim 1, wherein a plurality of probe sets and/or a plurality of libraries and/or a plurality of sequencing techniques is used to obtain a plurality of first computer readable files, and further comprising a step of:
f1. Selecting among said plurality of first computer readable file at least one first computer readable file corresponding to the highest genotypability information.

3. Method according to claim 2, wherein at least a second computer readable file including non-genotypability information of said selected genomic region is generated based on said selected at least one first computer readable file corresponding to the highest genotypability information.

4. Method according to claim 1, further comprising a step of: comparing the genomic coordinates of said selected genomic region with the genomic coordinates of said genotypable portions to obtain the genomic coordinates of said non-genotypable portions.

5. Method according to any previous claim, wherein in said step c. a plurality of libraries of nucleic acid fragments are provided, wherein each one of said libraries have a different predetermined average length of the fragments, and said step e. is carried out with a plurality of different sequencing techniques, to obtain a plurality of sequencing data pools, wherein each sequencing data pool relates to a determined combination of library and sequencing technique.

6. Method according to any previous claim 4 to 5, wherein said step of comparing the genomic coordinates of said selected genomic region with the genomic coordinates of said genotypable portions to obtain the genomic coordinates of said non-genotypable portions comprises a step of subtracting the genomic coordinates of said genotypable portions from said genomic coordinates of said genomic region.

7. Method according to any previous claim 1 to 6, further comprising step h. of producing a list of non-genotypable variants of said genomic region, said non-genotypable variants preferably having clinical relevance.

8. Method according to any previous claim, comprising a step of increasing said average length of said fragments from said first average length, to increase said genotypability information of said selected genomic region.

9. A method for the production of a kit for the enrichment of a selected genomic region, comprising a method according to any previous claim, and further comprising a step of including said probe set targeting said genomic region and said at least one first computer readable file including genotypability information of said selected genomic region and said at least one second computer-readable file including non genotypability information of said selected genomic region and/or said at least a list of non-genotypable variants of said genomic region, wherein said first computer-readable file is in gVCF format and said second computer-readable file is in BED format.

10. A kit for the enrichment of a selected genomic region in a genomic nucleic acid sample as obtainable with a method according to claim 9, comprising at least one probe set targeting said selected genomic region, at least one first computer readable file including genotypability information of said selected genomic region and at least one second computer-readable file including non genotypability information of said selected genomic region and/or at least a list of non-genotypable variants of said genomic region, wherein said genotypability information, said non-genotypability information and said a list of non-genotypable variants of said genomic region are based on the use of said at least one probe set in combination with a library having a predetermined fragments' length selected so that at least one flanking region of the selected genomic region is included in one or more fragments and wherein said library comprises fragments including at least a portion of an exon and at least a portion of an intron, and with the use of a predetermined sequencing technique, wherein said at least one first computer-readable file is in gVCF format and includes the genomic coordinates of the genotypable portions of said genomic region and wherein said at least one second computer-readable file is in BED format includes the genomic coordinates of non-genotypable portions of said genomic region.

11. Kit according to claim 10 further comprising reagents for the preparation of a sequencing library of nucleic acid fragments.

## Patentansprüche

1. Ein Verfahren zur Anreicherung einer ausgewählten genomischen Region, umfassend die Schritte:
a. Auswählen einer zu sequenzierenden genomischen Region;
b. Bereitstellen mindestens eines Sondensatzes, der auf die genomische Region abzielt;
c. Bereitstellen mindestens einer Bibliothek von Nukleinsäurefragmenten, wobei die Bibliothek mindestens die ausgewählte genomische Region umfasst, wobei die Fragmente eine vorbestimmte erste durchschnittliche Länge aufweisen, so dass mindestens eine flankierende Region der ausgewählten genomischen Region in einem oder in mehreren Fragmenten eingeschlossen ist, und wobei die Bibliothek Fragmente umfasst, die mindestens einen Abschnitt eines Exons und mindestens einen Abschnitt eines Introns umfassen;
d. Anreichern mindestens der genomischen Region in der Bibliothek durch den mindestens einen Sondensatz, um mindestens eine angereicherte ausgewählte genomische Region zu erhalten;
e. Sequenzieren der mindestens einen angereicherten ausgewählten genomischen Region unter Verwendung mindestens einer ersten Sequenzierungstechnik, um mindestens einen ersten Sequenzierungsdatenpool der ausgewählten genomischen Region zu erhalten;
f. Verarbeiten des mindestens ersten Sequenzierungsdatenpools, wobei mindestens eine erste computerlesbare Datei erhalten wird, die Genotypisierbarkeitsinformationen der ausgewählten genomischen Region enthält, wobei Schritt f. die Schritte umfasst:
f.a. Abgleichen des im Schritt e. erhaltenen Sequenzierungsdatenpools zu einer Referenzgenomsequenz;
f.b. Bestimmen genomischer Koordinaten genotypisierbarer Abschnitte der ausgewählten genomischen Region;
f.c. Erzeugen mindestens einer ersten computerlesbaren Datei, die als Genotypisierbarkeitsinformationen die genomischen Koordinaten der genotypisierbaren Abschnitte der ausgewählten genomischen Region enthält;
wobei die erste computerlesbare Datei im gVCF-Format vorliegt;
wobei das Verfahren weiterhin den Schritt umfasst:
g. Erzeugen mindestens einer zweiten computerlesbaren Datei, die Nicht-Genotypisierungsinformationen der ausgewählten genomischen Region enthält, basierend auf der mindestens ersten computerlesbaren Datei, wobei die zweite computerlesbare Datei als Nicht-Genotypisierbarkeitsinformationen die genomischen Koordinaten der nicht genotypisierbaren Abschnitte der ausgewählten genomischen Region enthält, und wobei die zweite computerlesbare Datei im BED-Format vorliegt.

2. Verfahren gemäß Anspruch 1, wobei mehrere Sondensätze und/oder mehrere Bibliotheken und/oder mehrere Sequenzierungstechniken verwendet werden, um mehrere erste computerlesbare Dateien zu erhalten, und ferner einen Schritt umfasst:
f1. Auswählen mindestens einer ersten computerlesbaren Datei, die den höchstwertigen Genotypisierbarkeitsinformationen entspricht, aus der Vielzahl von ersten computerlesbaren Dateien.

3. Verfahren gemäß Anspruch 2, wobei mindestens eine zweite computerlesbare Datei, die Nicht-Genotypisierbarkeitsinformationen der ausgewählten genomischen Region enthält, basierend auf der ausgewählten mindestens einen ersten computerlesbaren Datei erzeugt wird, die den höchstwertigen Genotypisierbarkeitsinformationen entspricht.

4. Verfahren gemäß Anspruch 1, ferner umfassend den folgenden Schritt:
Vergleichen der genomischen Koordinaten der ausgewählten genomischen Region mit den genomischen Koordinaten der genotypisierbaren Abschnitte, um die genomischen Koordinaten der nicht genotypisierbaren Abschnitte zu erhalten.

5. Verfahren gemäß einem der vorhergehenden Ansprüche, wobei in Schritt c. eine Vielzahl von Bibliotheken von Nukleinsäurefragmenten bereitgestellt werden, wobei jede der Bibliotheken eine andere vorbestimmte durchschnittliche Länge der Fragmente aufweist, und der Schritt e. mit mehreren unterschiedlichen Sequenzierungstechniken durchgeführt wird, um mehrere Sequenzierungsdatenpools zu erhalten, wobei sich jeder Sequenzierungsdatenpool auf eine bestimmte Kombination aus Bibliothek und Sequenzierungstechnik bezieht.

6. Verfahren gemäß einem der vorhergehenden Ansprüche 4 bis 5, wobei der Schritt des Vergleichens der genomischen Koordinaten der ausgewählten genomischen Region mit den genomischen Koordinaten der genotypisierbaren Abschnitte zum Erhalten der genomischen Koordinaten der nicht genotypisierbaren Abschnitte einen Schritt des Subtrahierens der genomischen Koordinaten der genotypisierbaren Abschnitte von den genomischen Koordinaten der genomischen Region umfasst.

7. Verfahren gemäß einem der vorhergehenden Ansprüche 1 bis 6, ferner umfassend Schritt h., der Erstellung einer Liste von nicht genotypisierbaren Varianten der genomischen Region, wobei die nicht genotypisierbaren Varianten vorzugsweise klinische Relevanz haben.

8. Verfahren gemäß einem der vorhergehenden Ansprüche, umfassend einen Schritt des Erhöhens der durchschnittlichen Länge der Fragmente ausgehend von der ersten durchschnittlichen Länge, um die Genotypisierbarkeitsinformation der ausgewählten genomischen Region zu erhöhen.

9. Ein Verfahren zur Herstellung eines Kits zur Anreicherung einer ausgewählten genomischen Region, umfassend ein Verfahren gemäß einem der vorhergehenden Ansprüche, und weiterhin umfassend einen Schritt, bei dem der Sondensatz auf die genomische Region abzielt, und die mindestens eine erste computerlesbare Datei die Genotypisierbarkeitsinformationen der ausgewählten genomischen Region enthält, und die mindestens eine zweite computerlesbare Datei die Nicht-Genotypisierbarkeitsinformationen der ausgewählten genomischen Region enthält und/oder die mindestens eine Liste von nicht genotypisierbaren Varianten der genomischen Region, wobei die erste computerlesbare Datei im gVCF-Format und die zweite computerlesbare Datei im BED-Format vorliegt.

10. Ein Kit zur Anreicherung einer ausgewählten genomischen Region in einer genomischen Nukleinsäureprobe, erhältlich mit einem Verfahren gemäß Anspruch 9, umfassend mindestens einen Sondensatz, der auf die ausgewählte genomische Region abzielt, mindestens eine erste computerlesbare Datei, die Genotypisierbarkeitsinformationen der ausgewählten genomischen Region enthält und mindestens eine zweite computerlesbare Datei, die Nicht-Genotypisierbarkeitsinformationen der ausgewählten genomischen Region enthält und/oder mindestens eine Liste von nicht genotypisierbaren Varianten der genomischen Region, wobei die Genotypisierbarkeitsinformationen, die Nicht-Genotypisierbarkeitinformationen und die Liste von nicht genotypisierbaren Varianten der genomischen Region auf der Verwendung des mindestens einen Sondensatzes in Kombination mit einer Bibliothek mit einer vorbestimmten Fragmentlänge beruhen, so ausgewählt, dass mindestens eine flankierende Region der ausgewählten genomischen Region in einem oder in mehreren Fragmenten eingeschlossen ist, und wobei die Bibliothek Fragmente umfasst, die mindestens einen Abschnitt eines Exons und mindestens einen Abschnitt eines Introns umfassen, und unter Verwendung einer vorbestimmten Sequenzierungstechnik, wobei die mindestens eine erste computerlesbare Datei im gVCF-Format vorliegt und die genomischen Koordinaten der genotypisierbaren Abschnitte der genomischen Region enthält, und wobei die mindestens eine zweite computerlesbare Datei im BED-Format vorliegt und die genomischen Koordinaten der nicht genotypisierbaren Abschnitte der genomischen Region enthält.

11. Kit gemäß Anspruch 10, ferner umfassend Reagenzien für die Herstellung einer Sequenzierungsbibliothek von Nukleinsäurefragmenten.

## Revendications

1. Procédé pour l'enrichissement d'une région génomique sélectionnée, comprenant les étapes consistant à :
a. Sélectionner une région génomique à séquencer ;
b. Fournir au moins un ensemble de sondes ciblant ladite région génomique ;
c. Fournir au moins une bibliothèque de fragments d'acide nucléique, ladite bibliothèque comprenant au moins ladite région génomique sélectionnée, dans lequel lesdits fragments ont une première longueur moyenne prédéterminée sélectionnée de sorte qu'au moins une région flanquante de la région génomique sélectionnée soit incluse dans un ou plusieurs fragments et dans lequel ladite bibliothèque comprend des fragments incluant au moins une partie d'un exon et au moins une partie d'un intron ;
d. enrichir au moins ladite région génomique dans ladite bibliothèque par l'intermédiaire dudit au moins un ensemble de sondes, pour obtenir au moins une région génomique sélectionnée enrichie ;
e. Séquencer ladite au moins une région génomique sélectionnée enrichie en utilisant au moins une première technique de séquençage, pour obtenir au moins un premier pool de données de séquençage de ladite région génomique sélectionnée ;
f. Traiter ledit au moins premier pool de données de séquençage en obtenant au moins un premier fichier lisible par ordinateur comprenant des informations de génotypabilité de ladite région génomique sélectionnée, dans lequel ladite étape f. comprend les étapes consistant à :
f.a. aligner le pool de données de séquençage obtenu dans ladite étape
e. sur une séquence génomique de référence ;
f.b. déterminer les coordonnées génomiques des parties génotypables de ladite région génomique sélectionnée ;
f.c. générer au moins un premier fichier lisible par ordinateur comprenant comme informations de génotypabilité les coordonnées génomiques des parties génotypables de ladite région génomique sélectionnée ;
dans lequel ledit premier fichier lisible par ordinateur est au format gVCF ;
ledit procédé comprenant en outre une étape consistant à :
g. Générer au moins un second fichier lisible par ordinateur comprenant des informations de non génotypabilité de ladite région génomique sélectionnée, sur la base dudit au moins premier fichier lisible par ordinateur, dans lequel ledit second fichier lisible par ordinateur comprend comme informations de non génotypabilité les coordonnées génomiques des parties non génotypables de ladite région génomique sélectionnée, et dans lequel ledit second fichier lisible par ordinateur est au format BED.

2. Procédé selon la revendication 1, dans lequel une pluralité d'ensembles de sondes et/ou une pluralité de bibliothèques et/ou une pluralité de techniques de séquençage est utilisée pour obtenir une pluralité de premiers fichiers lisibles par ordinateur, et comprenant en outre une étape consistant à :
f1. Sélectionner parmi ladite pluralité de premiers fichiers lisibles par ordinateur au moins un premier fichier lisible par ordinateur correspondant aux informations de génotypabilité la plus élevée.

3. Procédé selon la revendication 2, dans lequel au moins un second fichier lisible par ordinateur comprenant des informations de non génotypabilité de ladite région génomique sélectionnée est généré sur la base dudit au moins un premier fichier lisible par ordinateur sélectionné correspondant aux informations de génotypabilité les plus élevées.

4. Procédé selon la revendication 1, comprenant en outre une étape consistant à : comparer les coordonnées génomiques de ladite région génomique sélectionnée avec les coordonnées génomiques desdites parties génotypables pour obtenir les coordonnées génomiques desdites parties non génotypables.

5. Procédé selon l'une quelconque des revendications précédentes, dans lequel dans ladite étape c. une pluralité de bibliothèques de fragments d'acide nucléique sont fournies, dans lequel chacune desdites bibliothèques a une longueur moyenne prédéterminée différente des fragments, et ladite étape e. est réalisée avec une pluralité de techniques de séquençage différentes, pour obtenir une pluralité de pools de données de séquençage, dans lequel chaque pool de données de séquençage se rapporte à une combinaison déterminée de bibliothèque et de technique de séquençage.

6. Procédé selon l'une quelconque des revendications précédentes 4 à 5, dans lequel ladite étape de comparaison des coordonnées génomiques de ladite région génomique sélectionnée avec les coordonnées génomiques desdites parties génotypables pour obtenir les coordonnées génomiques desdites parties non génotypables comprend une étape de soustraction des coordonnées génomiques desdites parties génotypables desdites coordonnées génomiques de ladite région génomique.

7. Procédé selon l'une quelconque des revendications précédentes 1 à 6, comprenant en outre l'étape h. de production d'une liste de variants non génotypables de ladite région génomique, lesdits variants non génotypables ayant de préférence une pertinence clinique.

8. Procédé selon l'une quelconque des revendications précédentes, comprenant une étape consistant à augmenter ladite longueur moyenne desdits fragments à partir de ladite première longueur moyenne, afin d'augmenter lesdites informations de génotypabilité de ladite région génomique sélectionnée.

9. Procédé de production d'un kit pour l'enrichissement d'une région génomique sélectionnée, comprenant un procédé selon l'une quelconque des revendications précédentes, et comprenant en outre une étape consistant à inclure ledit ensemble de sondes ciblant ladite région génomique et ledit au moins un premier fichier lisible par ordinateur comprenant des informations de génotypabilité de ladite région génomique sélectionnée et ledit au moins un second fichier lisible par ordinateur comprenant des informations de non génotypabilité de ladite région génomique sélectionnée et/ou ladite au moins une liste de variants non génotypables de ladite région génomique, dans lequel ledit premier fichier lisible par ordinateur est au format gVCF et ledit second fichier lisible par ordinateur est au format BED.

10. Kit pour l'enrichissement d'une région génomique sélectionnée dans un échantillon d'acide nucléique génomique tel qu'il peut être obtenu avec un procédé selon la revendication 9, comprenant au moins un ensemble de sondes ciblant ladite région génomique sélectionnée, au moins un premier fichier lisible par ordinateur comprenant des informations de génotypabilité de ladite région génomique sélectionnée et au moins un second fichier lisible par ordinateur comprenant des informations de non génotypabilité de ladite région génomique sélectionnée et/ou au moins une liste de variants non génotypables de ladite région génomique, dans lequel lesdites informations de génotypabilité, lesdites informations de non-génotypabilité et ladite liste de variants non génotypables de ladite région génomique sont basées sur l'utilisation dudit au moins un ensemble de sondes en combinaison avec une bibliothèque ayant une longueur de fragments prédéterminée sélectionnée de sorte qu'au moins une région flanquante de la région génomique sélectionnée soit incluse dans un ou plusieurs fragments et dans lequel ladite bibliothèque comprend des fragments incluant au moins une partie d'un exon et au moins une partie d'un intron, et avec l'utilisation d'une technique de séquençage prédéterminée, dans lequel ledit au moins un premier fichier lisible par ordinateur est au format gVCF et comprend les coordonnées génomiques des parties génotypables de ladite région génomique et dans lequel ledit au moins un second fichier lisible par ordinateur est au format BED comprend les coordonnées génomiques des parties non génotypables de ladite région génomique.

11. Kit selon la revendication 10, comprenant en outre des réactifs pour la préparation d'une bibliothèque de séquençage de fragments d'acide nucléique.
